# EUROPEAN PATENT APPLICATION

(11) **EP 3 827 869 A1**
(43) Date of publication of application: **02.06.2021**
(21) Application number: 19854518.8
(22) Date of filing: 22.07.2019
(51) Int. Cl.: A61M 37/00, A61K 8/02, A61K 8/60, A61K 8/81, A61K 8/85, A61K 8/90, A61K 9/70, A61K 31/7028, A61K 47/32, A61K 47/34, A61M 35/00, A61P 17/00, A61P 43/00, A61Q 19/00, A61Q 19/08

(54) **SKIN QUALITY-IMPROVING SHEET**

(30) Priority: 28.08.2018 JP 2018159100
(71) Applicant: Nissha Co., Ltd., Kyoto-shi, Kyoto 604-8551 (JP)
(72) Inventor: Hashimoto, takao, Kyoto-shi, Kyoto 604-8551 (JP)
(74) Representative: Zimmermann, Tankred Klaus
(86) International application number: PCT/JP2019/028592
(87) International publication number: WO 2020/044853

(57) **Abstract**

An object is to provide a skin modifying sheet as described below. According to this skin modifying sheet, a period until effects of ethyl-α-D-glucoside appear is shorter than when food products or cosmetics containing ethyl-α-D-glucoside are orally taken in or applied on the skin, and collagen in the skin can be increased at a pinpoint location. The skin modifying sheet includes a sheet-like substrate and a plurality of fine needles which is formed on one surface of the sheet-like substrate and contains ethyl-α-D-glucoside.

## Description

### TECHNICAL FIELD

The present invention relates to a skin modifying sheet.

### BACKGROUND ART

In recent years, ethyl-α-D-glucoside (hereinafter, sometimes described as α-EG) attracts attention as an ingredient to increase the collagen amount in the skin (for example, see Non-Patent Literature 1). α-EG is an ingredient which is mainly contained in refined sake, sweet sake, sake lees, moromi mash, and the like. When food products and the like containing α-EG are orally taken in or when cosmetics such as creams containing α-EG (for example, see Patent Literature 1) are applied on the skin, the α-EG permeates the dermis in the skin, and fibroblasts present in the dermis are activated. Fibroblasts exist near capillary vessels of the dermis and can produce collagen and the like. When fibroblasts are activated by α-EG, the production amount of collagen in the skin increases, and resilience of the skin can be improved.

### CITATION LIST

### PATENT LITERATURE

PATENT LITERATURE 1: JP-A-2010-115169

### NON-PATENT LITERATURE

Non-Patent Literature 1: "World's first. A study group of Kenji Ozeki lab in Kanazawa Institute of Technology and Shata Shuzo Co., Ltd. academically demonstrated that Japanese sake's umami ingredient 'α-EG' increases the collagen amount of the skin dermis layer. Resilient skin is kept for several weeks in proper amounts. It was also found that the effects are significant for aged women." [online] September 13, 2017, Kanazawa Institute of Technology, [searched on August 3, 2018], Internet <URL: https://www.kanazawa-it.ac.jp/kitnews/2017/0913_ozeki.html?_ga=2.165548983.903370888.1533287782-1270136493.1533287782>

### SUMMARY OF THE INVENTION

### PROBLEMS TO BE SOLVED BY THE INVENTION

However, the problem was that when food products and the like containing α-EG are orally taken in, it takes a long period (for examples, 6 weeks or more) until the effects by α-EG appear. Another problem was that collagen cannot be increased at a pinpoint location where resilience is desired to improve. On the other hand, when cosmetics containing α-EG are applied on the skin, the problem was that since α-EG is unlikely to permeate deeply in the skin, the effects significantly decrease.

The present invention has been made for solving the above-described problems. An object of the present invention is to provide a skin modifying sheet which can efficiently improve resilience of the skin at a pinpoint location.

### SOLUTION TO PROBLEMS

Hereinafter, a plurality of aspects will be described as a solution to problems. These aspects can be optionally combined as necessary.

A skin modifying sheet according to the present invention includes: a sheet-like substrate; and a plurality of fine needles which is formed on one surface of the sheet-like substrate and contains ethyl-α-D-glucoside.

Also, a plurality of the fine needles may have a shape that sticks in skin and a length that reaches dermis of skin.

Also, a plurality of the fine needles may have a shape that sticks in skin and a length that remains inside epidermis of skin.

Also, a plurality of the fine needles may have a length that remains inside a horny layer of the epidermis.

Also, a plurality of the fine needles may have a shape in which only a tip portion sticks in skin, the tip portion may have a length that remains inside a horny layer of skin, and a portion other than the tip portion may have a length that presses and elongates a surface of the horny layer.

Also, a plurality of the fine needles may have a shape that does not stick in skin and a length that presses and elongates a horny layer surface of skin.

### EFFECTS OF INVENTION

The skin modifying sheet of the present invention includes a sheet-like substrate and a plurality of fine needles which is formed on one surface of the sheet-like substrate and contains ethyl-α-D-glucoside.

Therefore, according to the present invention, there can be provided a skin modifying sheet which can efficiently improve resilience of the skin at a pinpoint location.

### BRIEF DESCRIPTION OF THE DRAWINGS

Fig. 1(a) is a schematic perspective view illustrating an embodiment of the skin modifying sheet of the present invention. Fig. 1(b) is an A-A cross-sectional view of Fig. 1(a).
Figs. 2(a) to 2(e) are schematic perspective views illustrating examples of the shape of a fine needle.
Figs. 3(a) and 3(b) are schematic cross-sectional views for explaining a relationship between the fine needle according to Variation Example 1 and the skin.
Fig. 4 is a schematic cross-sectional view for explaining a relationship between the fine needle according to Variation Example 2 and the skin.
Fig. 5 is a schematic cross-sectional view for explaining a relationship between the fine needle according to Variation Example 3 and the skin.
Fig. 6 is a schematic cross-sectional view illustrating an example of the shape of the fine needle according to Variation Example 4.
Figs. 7(a) and 7(b) are schematic cross-sectional views for explaining a relationship between the fine needle according to Variation Example 4 and the skin.
Fig. 8 is a schematic cross-sectional view illustrating an example of the shape of the fine needle according to Variation Example 5.
Figs. 9(a) and 9(b) are schematic cross-sectional views for explaining a relationship between the fine needle according to Variation Example 5 and the skin.
Figs. 10(a) to 10(d) are schematic cross-sectional views illustrating an example of the production method of the skin modifying sheet of the present invention.
Figs. 11(a) to 11(c) are schematic cross-sectional views illustrating an example of the production method of the skin modifying sheet of the present invention.

### DESCRIPTION OF EMBODIMENTS

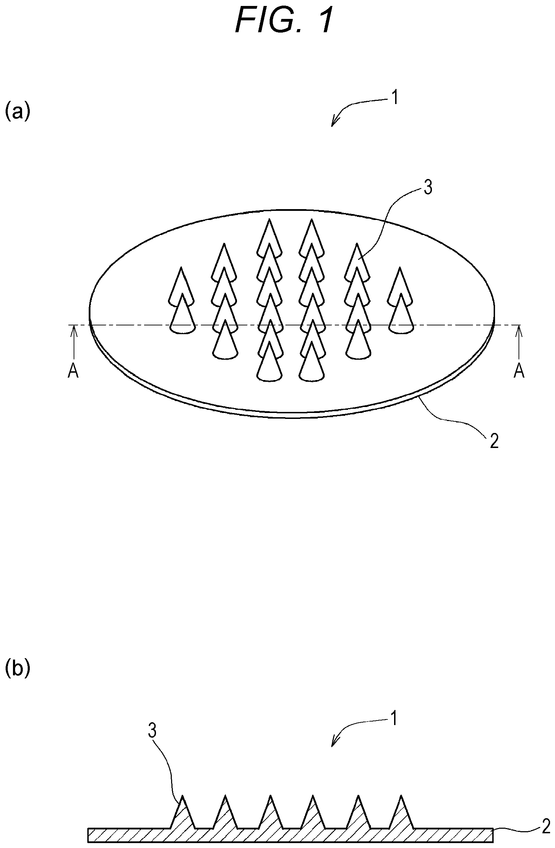

An example of the embodiment of the skin modifying sheet of the present invention will be described. A skin modifying sheet 1 of the present invention includes a sheet-like substrate 2 and a plurality of fine needles 3 which is formed on one surface of the sheet-like substrate 2 and contains ethyl-α-D-glucoside (see Fig. 1).

The sheet-like substrate 2 is formed in a varied planar shape in such a manner as to fit a site of the skin to which it is affixed. The thickness of the sheet-like substrate 2 may be, for example, 10 µm or more and 500 µm or less and preferably 20 µm or more and 200 µm or less, such that mechanical strength of the entire sheet can be ensured, and flexible deformation is enabled depending on the shape of the skin. The sheet-like substrate 2 may have either a single-layer structure constituted by a single material or a multi-layer structure formed from different materials.

The material of at least the front surface layer of the sheet-like substrate 2 is preferably the same as the material of the fine needles 3. The material of at least the front surface layer of the sheet-like substrate 2 is not particularly limited, as long as it can support the plurality of fine needles 3 in a state of being erected on the surface of the sheet-like substrate 2. Specifically, at least the front surface layer of the sheet-like substrate 2 is formed from a biologically harmless polymer substance. Examples of the biologically harmless polymer substance include biologically harmless resin, biologically harmless polysaccharides, and biologically harmless protein, as well as biologically harmless compounds derived therefrom. Here, being biologically harmless means being applicable for medical, cosmetic, or veterinary purposes when the use method is optimum, and the amount introduced to the skin is adequately adjusted.

The plurality of fine needles 3 is formed on one surface of the sheet-like substrate 2. The fine needles 3 are preferably formed from the same material as the material of the front surface layer of the sheet-like substrate 2. A material suitable for introducing α-EG as an intended substance into the skin is selected as a material of the fine needles 3. The fine needles 3 are also formed from a biologically harmless polymer substance. Examples of a polymer substance as a material of the fine needles 3 also include biologically harmless resin, biologically harmless polysaccharides, and biologically harmless protein, as well as biologically harmless compounds derived therefrom.

The biologically harmless polymer substance as a material of the fine needles 3 preferably has at least one property of biosolubility and biodegradability. Here, biosolubility is a property of being dissolved in vivo. Biodegradability is a property of being degraded in vivo. When the fine needles 3 are formed from a polymer substance having at least one of both the properties, the fine needles 3 which have invaded the skin gradually change in vivo with time under at least one action of dissolution and degradation. Therefore, the fine needles 3 in a solid form do not remain in the skin for a long period.

Also, the biosoluble and/or biodegradable polymer substance constituting the fine needles 3 is preferably water-soluble. When the fine needles 3 which have invaded the skin are a water-soluble polymer substance, the fine needles 3 dissolve by moisture existing in the skin. This facilitates smooth introduction of α-EG as an intended substance into the skin. Examples of the biologically harmless saccharides which are water-soluble and have at least one property of biosolubility and biodegradability and the biologically harmless compounds derived therefrom include maltose, dextran, water-soluble chitosan, pullulan, chondroitin sulphate sodium, sodium hyaluronate, and glycogen. Examples of the biologically harmless protein which are water-soluble and have at least one property of biosolubility and biodegradability and the biologically harmless compounds derived therefrom include serum albumin and serum α acid glycoprotein. Examples of the biologically harmless resin which are water-soluble and have at least one property of biosolubility and biodegradability and the biologically harmless compounds derived therefrom include water-soluble biologically harmless biodegradable polymers and compounds derived therefrom. Examples of the water-soluble biologically harmless biodegradable polymers and the compounds derived therefrom include a carboxylic vinyl polymer and a block polymer having water-solubility and biodegradability. In this block polymer, polyethylene glycol (PEG) which is a water-soluble and biocompatible polymer and polylactic-co-glycolic acid (PLGA), polycaprolactone (PCL), or polylactic acid (PLA) are block-copolymerized. Also, examples of the biologically harmless resin which are water-insoluble and have at least one property of biosolubility and biodegradability and the biologically harmless compounds derived therefrom include polylactic acid, polyglycolic acid, and polydioxanone.

The above-described materials used in the fine needles 3 can also be used as a material of the sheet-like substrate 2.

α-EG can be produced by a known method such as concentrating refined sake. For example, α-EG can be efficiently produced by a method disclosed in JP-A-2002-125692. That is, this method is a method of producing α-EG by causing transglucosidase derived from Talaromyces duponti to act on saccharides in the presence of ethanol. The resulting α-EG is white powder. This can be used by, for example, dissolving in water and a small amount of ethanol.

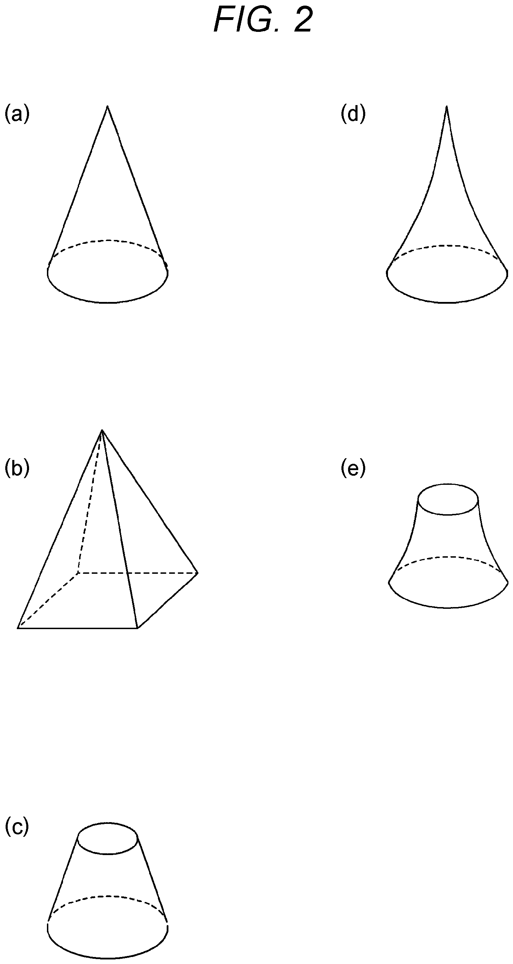

Examples of the shape of the fine needles 3 can be cone (see Fig. 2(a)), pyramid (see Fig. 2(b)), truncated cone (see Fig. 2(c)), and Konide (see Figs. 2(d) and 2(e)). The Konide shape represents a shape in which the side surface of a cone shape or a truncated cone shape is inwardly curved. The projection height of the fine needles 3 from the sheet-like substrate 2 can be, for example, several tens of µm to several mm. The density of the fine needles 3 can be, for example, 100 needles/cm² to 500 needles/cm².

The skin modifying sheet 1 of the present invention is formed from a polymer substance having at least one property of biosolubility and biodegradability. The plurality of fine needles 3 contains α-EG as an intended substance. When the skin modifying sheet 1 is affixed on, for example, the face, the polymer substance is dissolved and/or degraded in the skin, and α-EG can be directly introduced into the skin. When the introduced α-EG permeates into the dermis, fibroblasts in the dermis layer are activated, and the production amount of collagen can be increased. Therefore, since α-EG is directly introduced in the skin through the fine needles 3 of the skin modifying sheet of the present invention, the collagen amount can be increased at a pinpoint location for a shorter period than when α-EG is orally taken in. Also, since α-EG is directly introduced into the skin through the fine needles 3, α-EG can permeate the skin more efficiently than when α-EG is applied on the skin. Therefore, the collagen amount can be increased at a pinpoint location.

### (Variation Example 1)

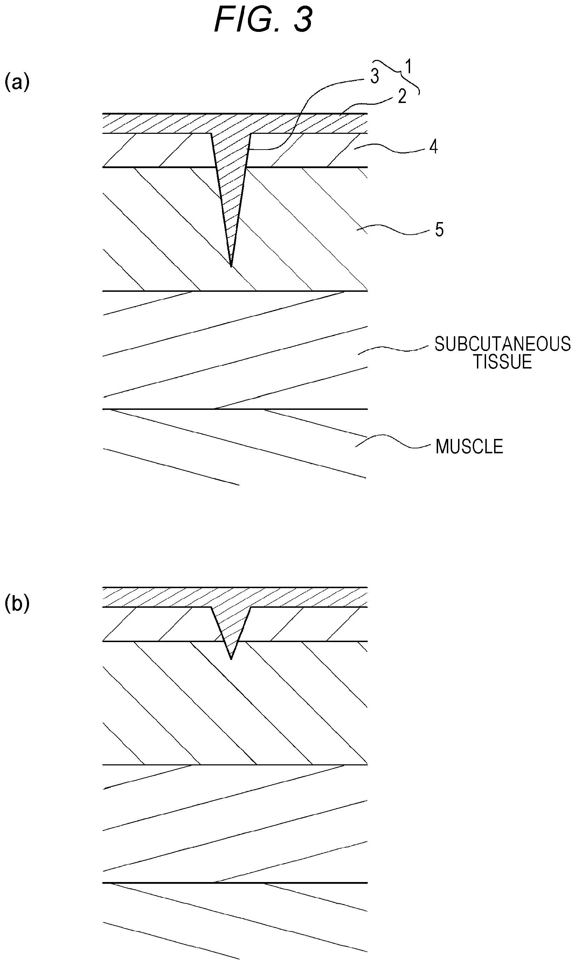

The skin modifying sheet 1 according to Variation Example 1 of the above-described embodiment will be described. In the skin modifying sheet 1 according to Variation Example 1, the plurality of fine needles 3 has a shape that sticks in the skin and a length that reaches a dermis 5 of the skin (see Fig. 3). The shape of the fine needles 3 is not particularly limited, as long as it is a shape that sticks in the skin. Examples of this shape include shapes illustrated in Figs. 2(a), 2(b), and 2(d). The length of the fine needles 3 is a length that reaches the dermis 5. An epidermis 4 exists on the dermis 5. The thickness of the epidermis 4 is at least 100 µm. Also, the thickness of the dermis 5 differs depending on the site but is about 3 mm at a maximum. Therefore, the length of the fine needles 3 of the skin modifying sheet 1 according to Variation Example 1 can be 100 µm to 3 mm. When the length is close to 3 mm, the fine needles 3 can reach a deep portion (a location close to the subcutaneous tissue) of the dermis 5, as illustrated in Fig. 3(a). Also, when the length is close to 100 µm, the fine needles 3 can reach a location where it penetrates through the epidermis 4, as illustrated in Fig. 3(b).

In the skin modifying sheet 1 according to Variation Example 1, the plurality of fine needles 3 containing α-EG reaches the dermis 5. The fine needles 3 are dissolved and/or degraded in the dermis 5 to release α-EG to the dermis 5 in a sustained manner. Therefore, fibroblasts present in the dermis 5 can be activated more reliably, and collagen can be increased for a shorter period and more efficiently.

### (Variation Example 2)

The skin modifying sheet 1 according to Variation Example 2 of the above-described embodiment will be described. In the skin modifying sheet 1 according to Variation Example 2, the plurality of fine needles 3 has a shape that sticks in the skin and a length that remains inside the epidermis 4 of the skin. The fine needles 3 can have the same shape as in Variation Example 1. The length of the fine needles 3 is a length that remains inside the epidermis 4. The thickness of the epidermis 4 is 100 µm to 300 µm. Therefore, the length of the fine needles 3 of the skin modifying sheet 1 according to Variation Example 2 can be 100 µm to 300 µm.

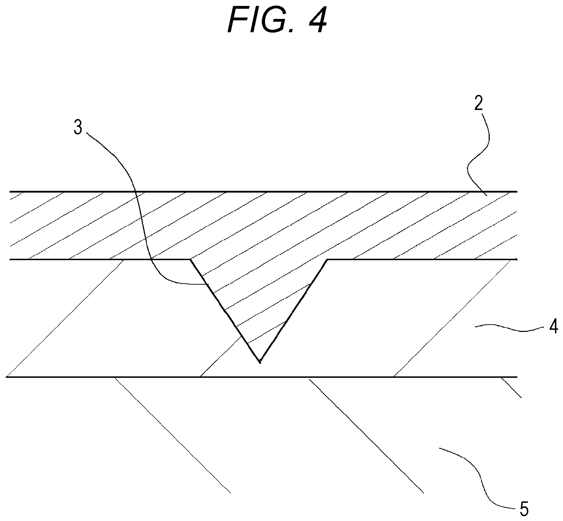

In the skin modifying sheet 1 according to Variation Example 2, the fine needles 3 containing α-EG can remain closer to the dermis 5 than when cosmetics containing α-EG are applied (see Fig. 4). Therefore, α-EG contained in the fine needles 3 permeates the dermis 5 more easily. When α-EG which has permeated the dermis 5 activates fibroblasts in the dermis 5, the production amount of collagen can be increased for a short period and efficiently.

### (Variation Example 3)

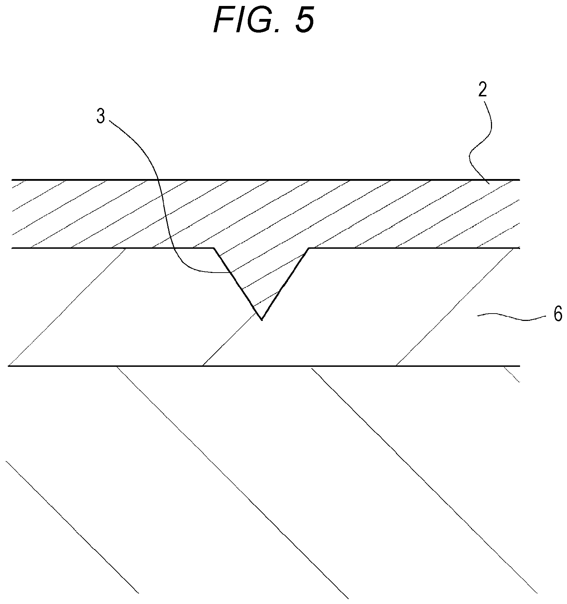

The skin modifying sheet 1 according to Variation Example 3 of the above-described embodiment will be described. In the skin modifying sheet 1 according to Variation Example 3, the plurality of fine needles 3 has a length that remains inside a horny layer 6 of the epidermis 4 (see Fig. 5). The fine needles 3 can have the same shape as in Variation Example 2. The thickness of the horny layer 6 is 1 µm to 20 µm. Therefore, the length of the fine needles 3 of the skin modifying sheet 1 according to Variation Example 3 can be 1 µm to 20 µm. However, about 1/10 of the length of the needle can be pressed in by normal finger pressure. Therefore, the length of the fine needles is desirably 50 µm to 200 µm.

In the skin modifying sheet 1 according to Variation Example 3, the fine needles 3 containing α-EG can remain inside the horny layer to release α-EG in a sustained manner. Therefore, α-EG can be likely to permeate the dermis more efficiently than when cosmetics containing α-EG are applied. When α-EG which has permeated the dermis 5 activates fibroblasts in the dermis 5, the production amount of collagen can be increased for a short period and efficiently.

### (Variation Example 4)

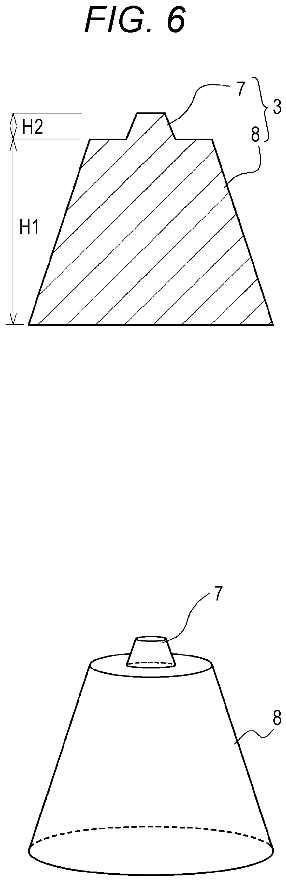

The skin modifying sheet 1 according to Variation Example 4 of the above-described embodiment will be described. In the skin modifying sheet 1 according to Variation Example 4, the plurality of fine needles 3 has a shape in which only a tip portion sticks in the skin. A portion other than the tip portion has a length that presses and elongates the horny layer surface. The shape of the fine needles 3 can be, for example, a shape disclosed in Japanese Patent No. 6023752. That is, the fine needles 3 have a shape that includes a truncated cone-shaped skin elongating portion 8 and a thorn-like projection 7 (an example of the tip portion) formed on the tip surface of the skin elongating portion 8 (see Fig. 6). The height H1 of the skin elongating portion 8 can be 30 µm to 300 µm. The height H2 of the thorn-like projection 7 can be 1 µm to 20 µm. However, about 1/10 of the height of the thorn-like projection can be pressed in by normal finger pressure. Therefore, the height of the thorn-like projection is desirably 50 µm to 200 µm.

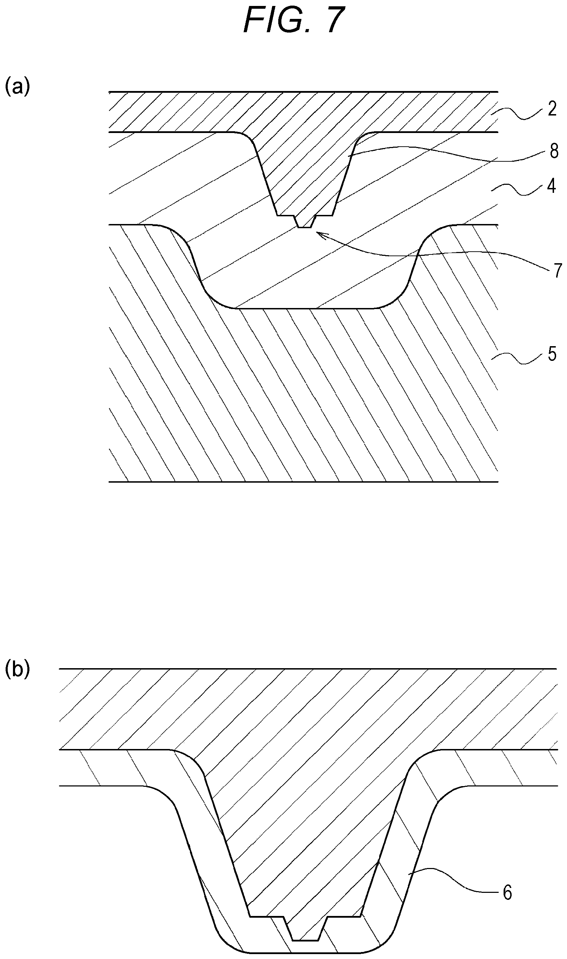

When the fine needles 3 having the thorn-like projection 7 are affixed on the skin, the skin around the skin elongating portion 8 elongates so that the skin surface reaches the surface of the sheet-like substrate 2, and the thorn-like projection 7 sticks in the horny layer 6 (see Figs. 7(a) and 7(b)). The skin elongating portion 8 does not stick in the horny layer 6.

In the skin modifying sheet 1 according to Variation Example 4, the thorn-like projection 7 as the tip portion sticks in the horny layer 6, and α-EG contained in the thorn-like projection 7 is released in a sustained manner into the horny layer 6. Also, α -EG is released in a sustained manner into the horny layer 6 from the tip surface and the side surface of the skin elongating portion 8. Therefore, α-EG can permeate into the horny layer more efficiently than when cosmetics containing α-EG are applied on the skin.

Also, in the skin modifying sheet 1 according to Variation Example 4, the tip surface of the skin elongating portion 8 does not penetrate through the horny layer and remains on the horny layer surface. Therefore, the protection function of the horny layer can be prevented from decreasing.

### (Variation Example 5)

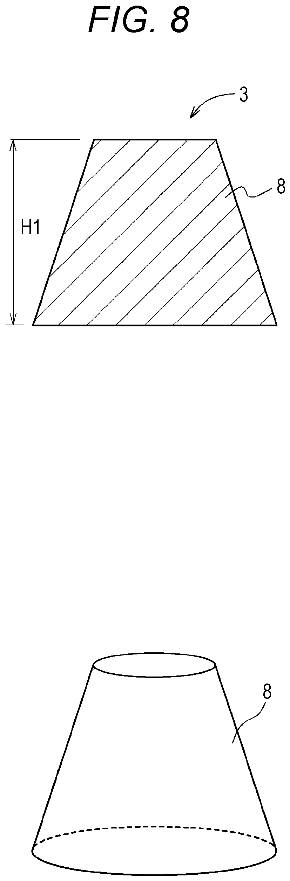

The skin modifying sheet 1 according to Variation Example 5 of the above-described embodiment will be described. In the skin modifying sheet 1 according to Variation Example 5, the plurality of fine needles 3 has a shape that does not stick in the skin and a length that presses and elongates the horny layer surface of the skin. The shape of the fine needles 3 is not particularly limited, as long as it is a shape that does not stick in the skin. For example, this shape can be a shape disclosed in Japanese Patent No. 6023752. That is, the fine needles 3 has a shape that includes only the truncated cone-shaped skin elongating portion 8 (see Fig. 8). The height H1 of the skin elongating portion 8 can be 30 µm to 300 µm.

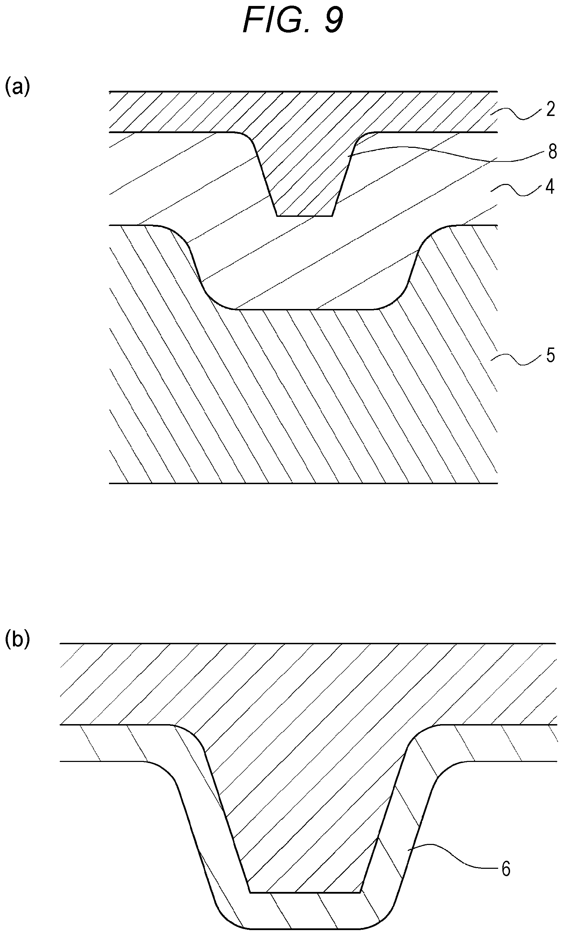

When the fine needles 3 having the skin elongating portion 8 are affixed on the skin, the skin around the skin elongating portion 8 elongates so that the skin surface reaches the surface of the sheet-like substrate 2 (see Figs. 9(a) and 9(b)). In the skin modifying sheet 1 according to Variation Example 5, the fine needles 3 do not stick in the horny layer 6, and α-EG is released in a sustained manner into the horny layer 6 from the tip surface and the side surface of the skin elongating portion 8. Therefore, α -EG can permeate into the horny layer more efficiently than when cosmetics containing α-EG are applied on the skin.

Also, in the skin modifying sheet 1 according to Variation Example 5, the tip surface of the skin elongating portion 8 does not penetrate through the horny layer and remains on the horny layer surface. Therefore, the protection function of the horny layer can be prevented from decreasing.

The skin modifying sheet 1 of the present invention can be produced by a known method which has been used in the past.

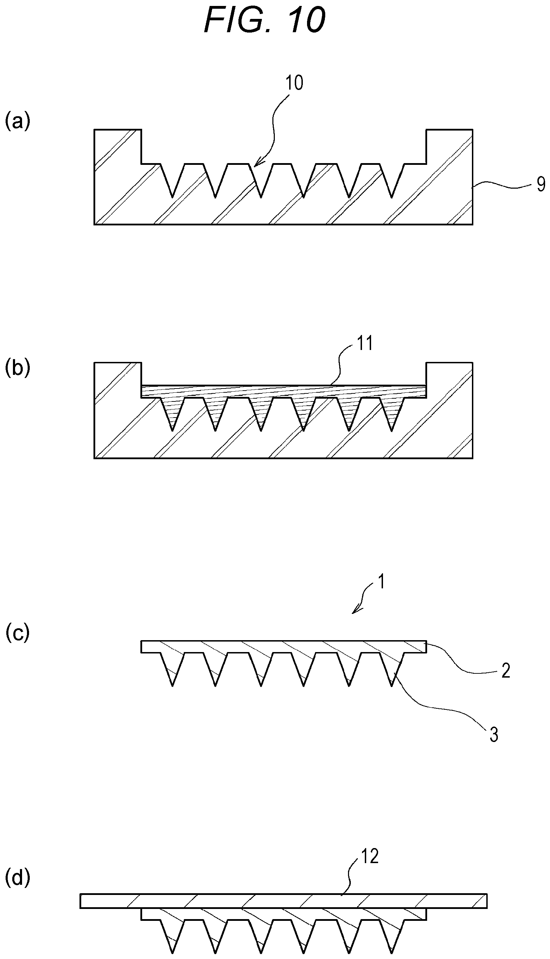

For example, a mold 9 including a plurality of concave portions 10 formed thereon is prepared (see Fig. 10(a)). As a material of the mold 9, a resin sheet, metal, or the like can be used. As a method of forming the concave portions 10, in a case of a resin sheet, a method of pressing a stamper (not illustrated) having the same shape as the fine needles 3 against a resin sheet may be used. In a case of metal, a method such as cutting or electric discharge machining may be used. Next, the concave portions 10 are filled with a raw material liquid 11 (see Fig. 10(b)). As a filling method, a method using a known apparatus such as an inkjet, a dispenser, a dispenser, or a squeegee can be used. At this time, filling with the raw material liquid 11 is continued until the concave portions 10 are filled, and furthermore the thickness of the sheet-like substrate 2 is obtained. After filling, drying is performed, and the skin modifying sheet 1 is peeled from the mold (see Fig. 10(c)).

It is noted that an adhesive sheet 12 may be bonded to the back surface of the skin modifying sheet 1, that is, to a surface of the sheet-like substrate 2 where the fine needles 3 are not formed (see Fig. 10(d)).

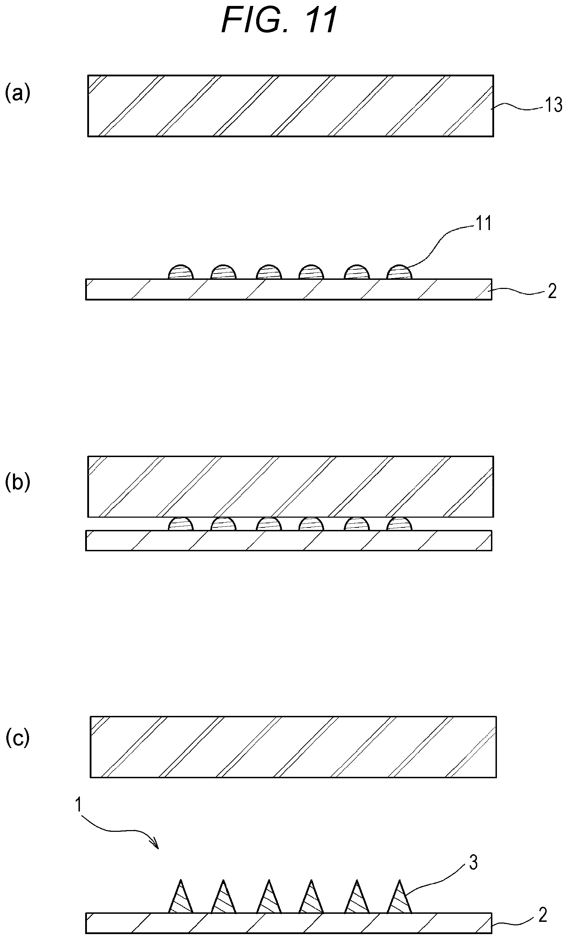

Alternatively, the following production method can also be used. First, the raw material liquid 11 is dropped on and attached to the sheet-like substrate 2 (see Fig. 11(a)). A plate 13 is moved closer to the raw material liquid 11 from a direction facing a surface to which the raw material liquid 11 was attached, such that the raw material liquid 11 is brought into contact with the plate 13 (see Fig. 11(b)). Thereafter, the plate 13 is gradually moved away from the sheet-like substrate 2 in a state parallel to the sheet-like substrate 2. Accordingly, the plurality of fine needles 3 is formed (see Fig. 11(c)). Therefore, the raw material liquid 11 preferably has a viscosity to a degree that it is stringy when the plate 13 is moved away.

### LIST OF REFERENCE SIGNS

1: skin modifying sheet
2: sheet-like substrate
3: fine needle
4: epidermis
5: dermis
6: horny layer
7: thorn-like projection
8: skin elongating portion
9: mold
10: concave portion
11: raw material liquid
12: adhesive sheet
13: plate

## Claims

1. A skin modifying sheet comprising:
a sheet-like substrate; and
a plurality of fine needles which is formed on one surface of the sheet-like substrate and contains ethyl-α-D-glucoside.

2. The skin modifying sheet according to claim 1,
wherein a plurality of the fine needles has a shape that sticks in skin and a length that reaches dermis of skin.

3. The skin modifying sheet according to claim 1,
wherein a plurality of the fine needles has a shape that sticks in skin and a length that remains inside epidermis of skin.

4. The skin modifying sheet according to claim 3,
wherein a plurality of the fine needles has a length that remains inside a horny layer of the epidermis.

5. The skin modifying sheet according to claim 1, wherein
a plurality of the fine needles has a shape in which only a tip portion sticks in skin,
the tip portion has a length that remains inside a horny layer of skin, and
a portion other than the tip portion has a length that presses and elongates a surface of the horny layer.

6. The skin modifying sheet according to claim 1,
wherein a plurality of the fine needles has a shape that does not stick in skin and a length that presses and elongates a horny layer surface of skin.
